# EUROPEAN PATENT APPLICATION

(11) **EP 1 356 819 A1**
(43) Date of publication of application: **29.10.2003**
(21) Application number: 02076675.4
(22) Date of filing: 25.04.2002
(51) Int. Cl.: A61K 38/22, A61K 38/55, A61P 3/00

(54) **Use of proteasome/ubiquitination inhibitors and/or MMP inhibitors alone or in combination with a growth hormone for treating /preventing catabolic conditions**

(71) Applicant: Universitair Medisch Centrum Utrecht, 3584 CX Utrecht (NL); Universiteit Utrecht, 3584 CX Utrecht (NL)
(72) Inventor: Strous, Gerardus Jacobus Antonius Maria, 4175 AP Haaften (NL); van Kerkhof, Petrus Johannes Maria, 6658 DB Beneden Leeuwen (NL)
(74) Representative: Prins, Adrianus Willem

(57) **Abstract**

The invention relates to the field of proteins, more specifically to proteins that are located on the surface of cells. The invention amongst others provides a method for up-regulating the bio-availability of a cell surface receptor comprising decreasing both the uptake and/or degradation of said receptor and the extracellular cleaving of said receptor. The invention further provides pharmaceutical compositions for up-regulating the bio-availability of a GHR comprising a proteasome/ubiquitination inhibitor and an MMP inhibitor and the use of said pharmaceutical compositions for the manufacture of a medicament for increasing anabolic conditions in human patients suffering from a catabolic state and/or cachexia.

## Description

The invention relates to the field of biology, more in particular to the field of medicine, more in particular to the treatment of a cachectic and/or a catabolic state in a patient caused by, for example, cancer, aids, severe burning uremia, Cushing's syndrome, neuromuscular disorders, immobility, and/or old age.

Growth hormone (GH) is essential for body growth in children. In adults, GH continues to stimulate many anabolic processes. The secretion of GH by the pituitary gland is pulsatile. For signal transduction, the presence of GH and GH receptors (GHR) is equally important. All body tissues contain the GHR. New insights have initiated new applications and a growing potential for GH replacement therapy in adults. In catabolic situations like cancer, aids, severe burning uremia, Cushing's syndrome, neuromuscular disorders and in persons that are bedridden, the activity of the GHR is decreased resulting in a loss of muscle tissue and skin proteins (wasting); the process of aging induces similar processes (reviewed in Mitch and Goldberg, 1996; Baumann, 2001).

The ubiquitin-system is involved in the degradation of the GHR (Strous and Govers, 1999; van Kerkhof et al., 2002). Selective degradation of many cytosolic proteins in eukaryotic cells is carried out by the ubiquitin system (Ciechanover and Schwartz, 2002). In this pathway, proteins are targeted for degradation by covalent ligation to ubiquitin. Ubiquitin-mediated degradation of regulatory proteins plays an important role in the control of numerous processes, including cell-cycle progression, signal transduction, transcriptional regulation, receptor down regulation, and endocytosis. The ubiquitin system has been implicated in immune response, development of the body, and programmed cell death. The specificity is guaranteed via a multitude of ubiquitin ligases (E3s), mostly multimodular protein complexes, which recognize target proteins and ligate ubiquitin moieties to the ε-NH2-group of lysine residues (Hershko et al., 2000). In muscle wasting the activity of the ubiquitin system is strongly increased, as reflected by increased levels of ubiquitin-conjugases, -ligases and proteasomes, while the synthesis of most other proteins is reduced (Mitch and Goldberg). The recognition that the system also regulates the residence time at the cell surface for receptors like for instance the GHR, opens new ways to control their activities. Apart from gene expression and mRNA stability, the number of GHRs per cell depends on its synthesis and quality control in the endoplasmic reticulum, removal of the GHR from the cell surface by proteases (shedding) and endocytosis, and the efficiency of targeting to lysosomes. Our recent experiments have shown that the availability of GHR for signal transduction upon GH binding is largely determined by the rate of uptake. GHR uptake is via clathrin-mediated endocytosis like for most cell surface receptors, e.g. transferrin, epidermal growth factor, insulin (van Kerkhof et al., 2002). A special feature of the GHR is that the ubiquitin system is required for uptake. A short intracellular amino acid motif is identified as a target for the ubiquitin system (Strous and Govers, 1999). When this motif is altered or when the ubiquitin system is switched off, the GHR residence time at the cell surface is prolonged 2-3-fold. The same effect would be anticipated by proteasome inhibitors. Treatment with proteasome inhibitors and/or ubiquitination inhibitors would therefore be beneficial for patients suffering from a catabolic state.

Recent studies have addressed the mechanisms involved in GHR shedding: removal of the extracellular GH-binding domain is accomplished by a metalloprotease, identified as tumor necrosis factor (TNF)-α-converting enzyme (ADAM 17 or TACE) (Zhang et al., 2000). The activity of this enzyme is increased by phorbol esters, presumably through a pathway involving the protein kinase C α (Amit et al., 2001). Phorbol ester-induced GHR proteolysis in 3T3-F442A and CHO-cells expressing rabbit GHR was significantly reduced by pretreatment with mitogen-activated protein (MAP) kinase inhibitors, suggesting involvement of the MAP kinase pathway (Takagi et al., 2001).

The present invention discloses that the ubiquitin/proteasome system is a negative regulator of this pathway. Thus, proteasome/ubiquitination inhibitors enhance the activity of TACE resulting in increased shedding of GHR from the cell membrane. As a consequence, the expected increase in residence time of GHR on the cell membrane, as a result of proteasome/ubiquitination inhibitors, is decreased by the enhanced activity of TACE. The net effect of proteasome/ ubiquitination inhibitors on the treatment of a catabolic state in patients is therefore less than expected.

Thus, there is a need for pharmacological tools or medication that can both up-regulate the number of GHR and/or prolong the residence time of GHR on a cell surface, for example to make a patient responding better to hormonal therapy or to provide the cells of said patient with a higher sensitivity to an endogenous hormone or other ligand.

The present invention provides a method for up-regulating the bio-availability of a cell surface receptor, for example growth hormone receptor (GHR), comprising decreasing both the degradation and the extracellular cleaving of said receptor. This is achieved by combining the actions of a proteasome/ubiquitination inhibitor and an MMP inhibitor. Surprisingly, this combination shows a synergistic effect on the increase in amount and residence time of the GHR on the cell membrane. In a preferred embodiment of the invention, the uptake in the cell, also known as endocytosis, following ubiquitin-ligation, and degradation in the lysosomes induced by the proteasome-ubiquitin system of said GHR is decreased by an inhibitor of the proteasome/ ubiquitination system, and at the same time the cleaving of said GHR on the outside of the cell membrane is decreased by an inhibitor of matrix metalloprotein. As a result, the number of available GHR on the cell membrane is increased and also the residence time of said GHR on the membrane is prolonged. Therefore, in a preferred embodiment this invention provides a method for up-regulating the bio-availability of a cell surface receptor, for example growth hormone receptor (GHR), comprising decreasing both the degradation of said receptor by the proteasome/ubiquitination system and the cleaving of said receptor by a matrix metalloprotein (MMP) inhibitor, for example, a TACE inhibitor. Of course the increase in the amount and residence of said GHR on the cell surface is optimal if both said inhibitors are bio available in the body at the same time. Bio available means that the inhibitor is present in such chemical or physical form as to be capable of being utilized by a living organism.

Therefore, in a preferred embodiment of the invention, a proteasome inhibitor is provided in sufficient amount in conjunction with sufficient amounts of an MMP inhibitor. In conjunction herein meaning that said proteasome/ubiquitination inhibitor is used or applied before, during or after administration of a sufficient amount of MMP inhibitor.

Several inhibitors of the proteasome/ubiquitination system are known in the art, like for example PS-341 and MG-132 and ubiquitin ligase inhibitor that work well in cells. Likewise, there is a number of effective MMP and Tumour Necrosis Factor Converting enzyme (TACE) inhibitors, like for example, Tapi-I and Marimastat. Now the mechanism is known, it is clear for a person skilled in the art, how to select, make, or produce, functional derivatives or analogues, that have the same effect at least in kind, if not in amount on the ubiquitination process, the degradation in the proteasome or the lysosome and the cleaving of GHR on the membrane. In a more preferred embodiment, said proteasome/ubiquitination inhibitor comprises a proteasome inhibitor and/or an ubiquitination inhibitor, for example, but not limited to PS-341, or MG-132 and said MMP inhibitor comprises a hydroxamic acid-based inhibitor, like for example a Tumour Necrosis Factor Converting enzyme (TACE) (also known as ADAM17) inhibitor, for example Tapi-I or Marimastat. The increasing effect of both said inhibitors on the amount and residence of said GHR on the cell surface increases an anabolic state which is beneficial for a patient in a cachectic or catabolic state. For signal transduction, both the GHR and growth hormone need to be present, thus the effect of both said inhibitors on the clinical results is increased by raising the level of growth hormone in said patient. Therefore, in another embodiment of the invention, said inhibitors are provided in conjunction to a growth hormone and/or a functional equivalent thereof, wherein functional equivalent of a growth hormone comprises any ligand that can exert a growth hormone like action and/or signal to the cell by binding to the GHR. Said growth hormone level can also be increased by administering substances that induce the release of natural growth hormone in the body. Therefore, in a more preferred embodiment of the invention, the growth hormone level in the body is intentionally increased, comprising administering a growth hormone releasing factor and/or a functional equivalent thereof and/or inhibiting a growth hormone release- inhibiting factor like somatostatin, wherein functional equivalent of a growth hormone releasing factor comprises any ligand that can exert a growth hormone releasing factor like action, and a functional equivalent of somatostatin comprises any ligand that can exert a somatostatin-like action. A functional derivative of a protein is defined as a protein which has been altered such that the properties of said protein are essentially the same in kind, not necessarily in amount. A derivative can be provided in many ways, for instance through conservative amino acid substitution.

A person skilled in the art is well able to generate analogous compounds of a protein. This can for instance be done through screening of a peptide library. Such an analogue has essentially the same immunogenic properties of said protein in kind, not necessarily in amount.

A proteasome/ubiquitination inhibitor and an MMP inhibitor can be combined in one composition, or they can be administered to the same patient in conjunction to each other. In conjunction herein meaning that said proteasome/ubiquitination inhibitor is used or applied before, during or after administration of MMP inhibitor. Thus one embodiment of the invention provides a pharmaceutical composition comprising a proteasome/ubiquitination inhibitor and an MMP inhibitor for up-regulating the bio-availability of a GHR. Another embodiment of the invention provides a pharmaceutical composition comprising a proteasome/ubiquitination inhibitor and an MMP inhibitor for up-regulating the bio-availability of a GHR, wherein both the inhibitors are combined before administering to a patient. As mentioned above the combination of both inhibitors with an increase of growth hormone is even more beneficial to a patient, because the increased amounts of GHR, together with growth hormone give a stronger and longer lasting signal to the cells. A more preferred embodiment of the invention thus provides said pharmaceutical composition for administering in conjunction with growth hormone or even more preferred in conjunction with growth hormone releasing factor. In conjunction herein meaning that said composition is used or applied before, during or after hormonal treatment with growth hormone and/or growth hormone releasing factor and up-regulates or modifies the activity of said hormone. The release of growth hormone can be inhibited by substances like for example somatostatin. Inhibiting said inhibitor, i.e. inhibiting for example somatostatin results in an increased release of growth hormone. Thus the increase of growth hormone can also be achieved by administration of a somatostatin inhibitor or a functional equivalent thereof. Therefore, the invention also provides said pharmaceutical composition for administering in conjunction with a somatostatin inhibitor or a functional equivalent or analogue thereof. All substances of abovementioned pharmaceutical compositions can be combined for an optimal treatment of patients, thus the invention also provides pharmaceutical compositions in which said inhibitors and/or growth hormone, and/or growth hormone releasing factor, and/or somatostatin is combined before administering to a patient. Treating patients with cachexia or a catabolic state with said pharmaceutical compositions results in an increased anabolic state of said patients. Thus, the invention also provides a method to use said pharmaceutical composition for the manufacture of a medicament for increasing anabolic conditions in patients suffering from a catabolic state and/or cachexia, wherein the cause of said catabolic state and/or cachexia comprises for example cancer, aids, severe burning, uremia, Cushing's syndrome, neuromuscular disorders, immobility and old age. Said medicament is also useful for the treatment of animals that are in a catabolic state. Therefore, another embodiment of the invention provides for said pharmaceutical compositions, said medicament and said methods to treat mammals suffering from a catabolic state and/or cachexia.

The invention is further explained in the following examples.

### Example 1

### Effect of proteasome and metalloprotease inhibitors on the GHR

### Materials and methods

*Cell lines and plasmids*. Chinese hamster lung cells, ts20, bearing a thermolabile ubiquitin-activating enzyme E1, and E36 control cells were stably transfected with a pCB6 construct containing the full length rabbit GHR cDNA sequence. The newly established cell lines were grown at 30°C in MEMα supplemented with 10% FCS, 4.5 g/l glucose, 100 U/ml penicillin, 100 µg/ml streptomycin and 0.45 mg/ml geneticin (van Kerkhof et al., 2002). Sixteen hours before the start of the experiment, 10 mM butyrate was added to increase the expression of the GHR (van Kerkhof et al., 2002).

*Antibodies and materials*. Polyclonal antibodies to the GHR cytosolic tail were raised in rabbits against fusion proteins of glutathione-S-transferase and GHR peptides consisting of amino acids 271-318 (anti-T) or 327-493 (anti-B) as described before (van Kerkhof et al., 2002). The antibody mAb5 recognising the lumenal part of the GHR was from AGEN Inc. (Parsippany, NJ). hGH was a gift from Lilly Research Labs (Indianapolis, IN). The metalloprotease inhibitors Tapi-I and GM6001, and the proteasome inhibitor MG-132 (carbobenzoxy-L-leucyl-L-leucyl-L-leucinal) were from Calbiochem. Inhibitors were dissolved in dimethylsulfoxide (DMSO).

*Ligand binding*. ¹²⁵I-human GH was prepared using chloramine T. Cells were washed with MEM" supplemented with 20 mM Hepes pH 7.4 and 0.1% BSA and incubated with ¹²⁵I-GH (8 nM) for 90 minutes at 0°C. Unbound radioactivity was aspirated, the cells were washed and the radioactivity was measured after solubilisation in 1 N NaOH. Non-specific radioactivity was determined in the presence of excess unlabelled ligand and subtracted.

*Cell lysis, immunoprecipitation, and western blotting.* Cells (in 6-cm dishes) were lysed on ice in 1.0 mL of lysis buffer containing 1% Triton X-100, 1 mM EDTA in PBS, with 50 mM NaF, 1 mM Na₃VO₄, 10 µg/ml aprotinin, 10 µg/ml leupeptin, and 1 mM PMSF. Cell lysates were subjected to SDS-polyacrylamide gel electrophoresis using a precast ready gel system (BioRad, Veenendaal, The Netherlands) and transferred to polyvinylidenedifluoride (PVDF) paper. The blots were immunostained using the polyclonal GHR antibodies anti-T or anti-B or the monoclonal antibody mAb5 followed by peroxidase conjugated protein A or rabbit anti-mouse IgG (Pierce Chemical Co.) as described previously (van Kerkhof et al. 2002), and detected by enhanced chemiluminescence (Roche Molecular Biochemicals).

### Results

### Effect of proteasome and metalloprotease inhibitors on the GHR.

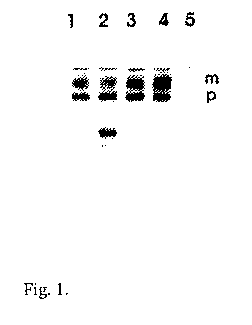

Proteasome inhibitors inhibit uptake of the GHR both in the presence and absence of ligand (van Kerkhof et al., 2002). The ubiquitin-dependent endocytosis (UbE) motif is required in both uptake modes (Strous and Govers, 1999; van Kerkhof et al., 2002). In the expectation that proteasome inhibitors would increase the amount of mature receptor we incubated the cells with MG132 for 3h. Surprisingly, the signal was decreased, while the amount of precursor (a measure for steady state synthesis) remained constant (Fig. 1, lane 2). Our previous studies showed that, once at the cell surface, the number of GHRs is also regulated by shedding (10-20%) (van Kerkhof et al., 2002).Therefore, we used the specific TACE inhibitor Tapi-I to examine its effect on the steady state concentration. As seen in Fig. 1, lane 3, this inhibitor induces a slight increase of the amount of mature GHR while the precursor form remained unchanged. In the next experiment (lane 4) we combined the two inhibitors: the steady state amounts of mature GHR was increased considerably as compared to control cells (lane 1). Again no change in the precursor form was detectable, indicating that inhibition of the proteasome had no effect on the steady-state amount of GHR in the ER. Lane 5 shows the effect of a 3-h period of protein synthesis inhibition by cycloheximide: this control experiment confirms that GHR has a high turnover and that indeed both species of the GHR have virtually disappeared from the biosynthetic compartment as well as at the cell surface. Thus, MG132 alone accelerates the GHR shedding considerably. This is also indicated by the appearance of an extra band of 65kD in lane 2, which is only detectable with antibodies against the cytosolic tail, representing the trans membrane and cytosolic tail of the GHR after removal of the GHBP by TACE.

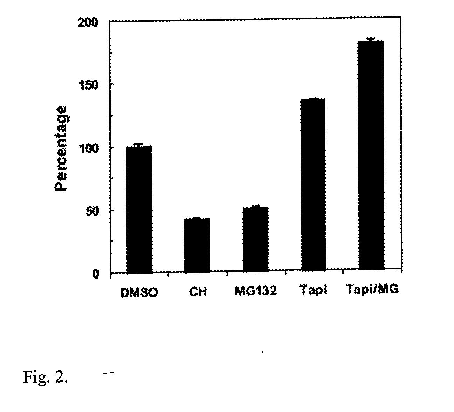

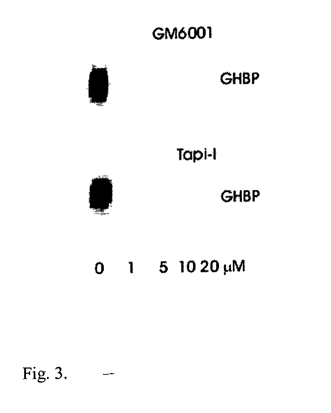

To quantify these results a binding study was performed using ¹²⁵I-GH (Fig. 2). The data show that the number of GHR at the cell surface decreased in the presence of MG132 to 50% of untreated cells, while a slight increase (35%) was detectable in the presence of a metalloprotease inhibitor. The combination of the two classes of inhibitors confirms the results of Fig. 2: an almost two-fold increase of GHR binding sites at steady state. Taken together: if the ubiquitin-proteasome system is inhibited by proteasome inhibitors, no GHR uptake occurs, but, due to the increased activity of TACE (increasing shedding), the net effect is less GHRs available for GH signal transduction. Our data show that application of a combination of proteasome inhibitors and TACE inhibitors results in a significant increase of the number of GHR at the cell surface. To illustrate that the effect of Tapi-I on the amount of mature GHRs is due to its effect on shedding, we examined the growth medium of the cells after 150 min for the presence of GH binding protein (GHBP). Western blot analysis confirmed both the general metalloprotease inhibitor GM6001 and the more specific TACE inhibitor Tapi-I almost completely inhibited the shedding of GHBP already at 1 µM. Hydroxamic acid-based inhibitors, which bind the essential zinc ion at the active site of the protease, were originally designed as inhibitors of zinc-dependent matrix metalloproteases (MMP). In addition, these inhibitors have been shown to inhibit protein ectodomain shedding of several different types of membrane proteins including pro-TNFα, Fas ligand, pro-transforming growth factor TGFα, 80-kDa TNFα receptor, IL-6 receptor, thyrotropin receptor, angiotensin converting enzyme, L-selectin, and β-APP,( Baumann, 2001). In this study, we used GM6001, a broad-spectrum matrix metalloprotease inhibitor to inhibit shedding, Tapi-I, an inhibitor of TACE, and MG132 to inhibit uptake. Based on our previous studies that the ubiquitin-proteasome system accounts for more than 80% of GHR disappearance from the cell surface via uptake and lysosomal degradation, we expected that MG132 activity would suffice to increase GHR at the cell surface at least two-fold. As the ubiquitin-proteasome system turns out to be a negative regulator of the MMPs, only the combination of the two inhibitors was effective in keeping the GHRs at the surface in the absence of GH. It should be noticed that the trimeric complex GH-GHR2 is resistant to MMP activity as we and others have published previously (van Kerkhof et al., 2002). Based on our data, we conclude that the combination of the two classes of drugs increases the availability of the GHR for GH approximately two-fold. Presently, prototypes of both class of inhibitors are being used in clinical trials to relieve/cure cancer in humans e.g. in treating patients with refractory or relapsed acute myeloid leukemia, acute lymphoblastic leukemia, chronic myeloid leukemia in blast phase, or myelodysplastic syndrome, and in a concomitant therapy with the potent proteasome inhibitor PS-341 and radiation in patients with recurrent or metastatic squamous cell carcinoma of the head and neck. Metalloprotease inhibitors, e.g. marimastat, are tested in many studies a.o. in patients with small cell lung cancer. As the GH-axis is a major regulator of metabolism throughout life, we use the potential of this drug combination to increase the number of GHRs at the cell surface which will induce anabolic conditions in humans suffering from catabolism and cachexia.

### Legends to the Figures.

Fig. 1.
   Effect of various drugs on GHR species. Cells were treated for 3 h as follows: lane 1, vehicle; lane 2, MG132 (10 µM); lane 3, Tapi-I (10 µM); lane 4, MG132 (10 µM) and Tapi-I (10 µM); lane 5, cycloheximide (0.1 mM); m, mature; p, precursor GHR. The lower band in lane 2 is the cytosolic tail, after removal of GHBP by TACE.
Fig. 2.
   Quantification of receptors at the cell surface. Cells were treated as in Fig. 1 with vehicle (DMSO), cycloheximide (CH), MG132, Tapi-I, and a combination of MG132 and Tapi-I (Tapi/MG) followed by incubation with ¹²⁵I-GH on ice. The number GHRs were calculated and expressed as a percentage of untreated cells.
Fig. 3.
   Effect of metalloprotease inhibitors on the release of GH binding protein. Cells grown in 12-cluster plates (0.15 mL MEM α/Hepes) were treated with increasing concentrations of the general MMP-inhibitor GM6001 or the specific TACE inhibitor Tapi-I. 15 µL samples were taken after 150 min at 30°C and analysed by Western blotting using Mab5 antibody.

### Reference List

Amit T, Hochberg Z, Yogev-Falach M, Youdim MB, Barkey RJ: Shedding of growth hormone-binding protein is inhibited by hydroxamic acid-based protease inhibitors: proposed mechanism of activation of growth hormone-binding protein secretase. J Endocrinol 169 (2001) 397-407.
Baumann G: Growth hormone binding protein 2001. J Pediatr Endocrinol Metab 14 (2001) 355-75.
Ciechanover A, Schwartz AL: Ubiquitin-mediated degradation of cellular proteins in health and disease. Hepatology 35 (2002) 3-6.
Hershko A, Ciechanover A, Varshavsky A: The ubiquitin system. Nature Medicine 6 (2000) 1073-1081.
Mitch WE, Goldberg AL: Mechanisms of disease: mechanisms of muscle wasting - the role of the ubiquitin-proteasome pathway. N Engl J. Med 335, (1996) 1897-1905.
Strous GJ, Govers R: The ubiquitin-proteasome system and endocytosis. J. Cell Science 112 (1999) 1417-1423.
Takagi K, Saito Y, Sawada J: Proteasome inhibitor enhances growth hormone-binding protein release. Mol Cell Endocrinol 182 (2001) 157-63.
van Kerkhof P, Smeets M, Strous GJ: The Ubiquitin-Proteasome Pathway Regulates the Availability of the GH Receptor. Endocrinol 143 (2002) 1243-1252.
Zhang Y, Jiang J, Black RA, Baumann G, Frank SJ: Tumor necrosis factor-α converting enzyme (TACE) is a growth hormone binding protein (GHBP) sheddase: the metalloprotease TACE/ADAM-17 is critical for (PMA-induced) GH receptor proteolysis and GHBP generation. Endocrinol 141 (2000) 4342-8.

## Claims

1. A method for up-regulating the bio-availability of a cell surface receptor comprising decreasing both the uptake and/or degradation of said receptor and the extracellular cleaving of said receptor.

2. A method according to claim 1 wherein said receptor comprises a growth hormone receptor (GHR).

3. A method according to claim 1 or 2 wherein said uptake and/or degradation is decreased by a proteasome/ubiquitination inhibitor.

4. A method according to any one of claims 1-3 wherein said cleaving is decreased by a matrix metalloprotein (MMP) inhibitor.

5. A method according to any one of claims 1-4 comprising providing a proteasome/ubiquitination inhibitor in conjunction with an MMP inhibitor.

6. A method according to any one of claims 1-5 wherein said proteasome/ubiquitination inhibitor comprises a proteasome inhibitor and/or an ubiquitination inhibitor, more specifically PS-341 or MG132 or a functional derivative or analogue thereof.

7. A method according to any one of claims 1-6 wherein said MMP inhibitor comprises a hydroxamic acid-based inhibitor or a functional derivative or analogue thereof

8. A method according to any one of claims 1-7 wherein said MMP inhibitor comprises a Tumour Necrosis Factor Converting enzyme (TACE) inhibitor or a functional derivative or analogue thereof.

9. A method according to any one of claims 1-8 wherein said MMP inhibitor comprises Tapi-I and/or Marimastat or a functional derivative or analogue thereof.

10. A method according to any of claims 1-9, wherein said inhibitors are provided in conjunction to a growth hormone and/or a functional equivalent thereof.

11. A method according to any of claims 1-10 also comprising increasing the growth hormone level in the body, comprising administering a growth hormone releasing factor and/or a functional equivalent thereof.

12. A method according to any of claims 1-11 also comprising increasing the growth hormone level in the body, comprising administering somatostatin and/or a functional equivalent thereof.

13. A pharmaceutical composition for up-regulating the bio-availability of a GHR comprising a proteasome/ubiquitination inhibitor and an MMP inhibitor.

14. A pharmaceutical composition according to claim 13 wherein said proteasome/ubiquitination inhibitor and said MMP inhibitor are combined before administering to a patient.

15. A pharmaceutical composition according to claim 13 or 14 also comprising a growth hormone and/or a functional equivalent thereof.

16. A pharmaceutical composition according to any one of claims 10-15 also comprising a growth hormone releasing factor and/or a functional equivalent thereof.

17. A pharmaceutical composition according to any one of claims 10-16 also comprising somatostatin and/or a functional equivalent thereof.

18. A pharmaceutical composition according to any one of claims 10-17, wherein said inhibitors and said growth hormone and/or said growth hormone releasing factor and/or said somatostatin and/or a functional equivalent of any of these, are combined before administering to a patient.

19. Use of a pharmaceutical composition according to any of claims 10-18 for the manufacture of a medicament for increasing anabolic conditions in human patients suffering from a catabolic state and/or cachexia.

20. Use according to claim 19, wherein the cause of said catabolic state and/or cachexia comprises a cancer, and/or an auto-immune disease, and/or an inflammatory disease.

21. Use of a pharmaceutical composition according to any of claims 13-18 for the manufacture of a medicament for increasing anabolic conditions in mammals suffering from a catabolic state and/or cachexia.
